# EUROPÄISCHE PATENTSCHRIFT

(11) **EP 0 873 758 B1**
(45) Veröffentlichungstag und Bekanntmachung des Hinweises auf die Patenterteilung: **16.10.2002**
(21) Anmeldenummer: 98107109.5
(22) Anmeldetag: 18.04.1998
(51) Int. Cl.: A61M 5/32

(54) **Nadelkappe für eine vorfüllbare Einmalspritze**
Needlecap for a pre-filled disposable syringe
Capuchon d'aiguille pour une seringue pré-remplie, à usage unique

(30) Priorität: 23.04.1997 DE 19717033
(43) Veröffentlichungstag der Anmeldung: 28.10.1998
(73) Patentinhaber: Schott Glas, 55122 Mainz (DE); CARL-ZEISS-STIFTUNG, 55122 Mainz (DE)
(72) Erfinder: Heinz, Jochen, Dr., 55578 Vendersheim (DE); Spallek, Michael, Dr., 55218 Ingelheim (DE)
(74) Vertreter: Fuchs Mehler Weiss & Fritzsche

(56) Entgegenhaltungen:
- EP-A- 0 429 052
- DE-A- 4 234 319
- DE-A- 4 438 360

## Beschreibung

Die Erfindung bezieht sich auf eine Einmalspritze mit einer im Spritzenkopf fest verbundenen Injektionsnadel und einer Nadelkappe, bestehend aus einer äußeren steifen Kunststoffkappe aus hartelastischem Material, die innen, zumindest im Bereich der Nadelspitze, mit einer aus einem weichelastischen Werkstoff bestehenden dichtenden Auskleidung versehen ist, und die einfach auf den Spritzenkopf aufstülpbar und ohne vorbereitenden Schritt von ihm vollständig abziehbar ist, wobei der Spritzenkopf und die steife Kunststoffkappe Elemente einer mechanisch stabilen form- und/oder kraftschlüssigen, den Aufstülpweg begrenzenden, Verbindungsanordnung besitzen, bei gleichzeitig mikrobiologisch sicheren Abdichtung im aufgesetzten Zustand der steifen Kunststoffkappe.

Vorgefüllte Einmalspritzen, auch Fertigspritzen genannt, seien sie aus Glas oder aus Kunststoff gefertigt, müssen während ihrer Lagerung zum Schutz der Injektionsnadel und des Spritzeninhaltes mit einer Nadelkappe versehen sein, die erst unmittelbar vor der Applikation entfernt wird. In Normen wird die Injektionsnadel auch als Injektionskanüle und die Nadelkappe als Schutzkappe bezeichnet.

Derartige Nadelkappen sind in den vielfältigsten Ausführungen bekannt bzw. auf dem Markt. Am weitesten verbreitet, sozusagen die klassische Ausführungsform, sind Nadelkappen, die aus einem Elastomer, vorzugsweise Gummi oder Naturkautschuk, hergestellt sind, wobei die Länge typischerweise so groß gewählt ist, daß die Spitze der Injektionsnadel beim Aufsetzen der Kappe in die Kappenspitze einsticht und von dem Material der Kappe dichtend umschlossen bleibt.

Eine derartige Nadelkappe in Verbindung mit einer im Spritzenkopf fest verbundenen Injektionsnadel ist beispielsweise durch die DE-27 17 830 A1 (dortige Fig. 6) und die DE- 44 38 360 A1 bekannt geworden. Auch die DE 4 234 319 A 1 zeigt eine Nadelkappe mit einem elastischen Kappenhemd, das sich an den Spritzenkopf anschmiegt. Derartige Nadelkappen besitzen auf der einen Seite die vorteilhafte Eigenschaft, daß sie einfach auf den Spritzenkopf aufstülpbar und von ihm ohne vorbereitende Schritte vollständig abziehbar ist, und dabei auch eine mikrobiologisch sichere Abdichtung gewährleistet, da sich das elastische Kappenhemd an den Spritzenkopf dichtend anschmiegt. Sie besitzt jedoch auf der anderen Seite folgende nachteilige Eigenschaften:
- Die Nadelkappe kann infolge der Weichheit des Werkstoffes nur bedingt mechanischen Beeinträchtigungen entgegenwirken. Sie kann bei der Handhabung durch den Anwender auch durchstochen werden, was eine nicht unerhebliche Verletzungsgefahr für den Anwender beinhaltet.
- Die Nadelkappe wird beim Abziehen zusammengedrückt und legt sich eng an die Kanülenoberfläche an. Dies führt zu einem, zumindest teilweise, Abstreifen des auf die Nadel aufgebrachten Silikons, das als Gleitmittel beim Injektionsvorgang dienen soll. Fehlt nun dieses Gleitmittel, ist die Injektion mit Schmerzen beim Injektionsvorgang verbunden. Ferner können sich Silikonpartikel/-tröpfchen bilden, die so in den Körper gelangen können.
- Die Montage der Nadelkappe ist schwierig, da eine sicher kontrollierbare Wegbegrenzung für den Aufstülpvorgang fehlt, so daß es leicht vorkommen kann, daß die Nadelkappe zu weit oder wenig weit auf den Spritzenkopf aufgesetzt wird. Beide Fälle sind äußerst nachteilig, da im ersten Fall die Nadel durch die Nadelkappe durchstechen kann, im zweiten Fall eine mikrobiologisch sichere Abdichtung nicht mehr gewährleitet ist.
- Die Injektionsnadel ist im aufgesetzten Zustand der Nadelkappe visuell durch den Anwender nicht kontrollier- bzw. beurteilbar, beispielsweise, ob die Nadel für den vorgesehenen Zweck die geeignete ist oder ob die Nadel verbogen, verunreinigt, beschädigt ist. Es muß dazu die Nadelkappe abgezogen und gegebenenfalls wieder aufgestülpt werden, was die große Gefahr der mikrobiologischen Kontamination der Nadel oder sogar des Spritzeninhalts bzw. ein Durchstechen der Nadelkappe und/oder ein Verbiegen der Nadel mit sich bringt.
- Durch das Abziehen der Nadelkappe wird ein Unterdruck an der Nadelspitze erzeugt, so daß es leicht zu einem Austreten der in die Spritze gefüllten Flüssigkeit kommt. Für den Anwender ist es notwendig, diesen Flüssigkeitstropfen zu entfernen, was zum einen die tatsächliche applizierte Dosis vermindert, zum anderen wird die äußere Nadeloberfläche mit Medikament benetzt. Bei der Injektion wird dadurch Medikament undefiniert in den Einstichkanal eingebracht, was generell unerwünscht ist und bei einigen Medikamenten, z. B. Antikogulanzien (Heparin etc.) zu Blutergüssen an der Einstichstelle führen kann.

Zur Verbesserung der mechanischen Eigenschaften derartiger Nadelkappen ist es durch die EP-0 429 052 bekannt, sie mit einem äußeren hartelastischen Schild zu versehen. Wenn auch derartige Nadelkappen gegen äußere mechanische Beeinträchtigungen besser geschützt sind und auch die Durchstechbarkeit von innen heraus und damit die erwähnte Verletzungsgefahr für den Anwender beseitigt sind, bleiben jedoch die anderen angeführten Nachteile - keine visuelle Kontrollier- und Beurteilungsmöglichkeit der Nadel im aufgesetzten Zustand der Nadelkappe, kein tropfenfreies Abziehen der Nadelkappe, Abstreifen der Nadelsilikonisierung sowie keine sichere Wegbegrenzung bei der Montage - bestehen.

Ein zusätzlicher Nachteil bei dieser Ausführung ist die Einschränkung der Möglichkeiten zur Sterilisierung der Nadeln bei aufgesetzter Nadelkappe: Eine Sterilisation mit energiereichen Lichtblitzen ist nicht möglich, ebenso verhindert der harte äußere Mantel eine für eine Dampfsterilisation ausreichende Dampfdurchlässigkeit dieser zweiteiligen Nadelkappe.

Ähnliche Nachteile bestehen bei der in der erwähnten DE- 44 38 360 A1 alternativ beschriebenen Nadelkappe, bestehend aus einer Kunststoffkappe aus relativ hartelastischem Material, die innen, zumindest im Bereich der Kanülenspitze, mit einem weichelastischen dichtenden Werkstoff ausgekleidet ist. Hierdurch kann dieser weichelastische Werkstoff auf optimale Dichtungseigenschaften ausgewählt sein (Dichtfunktion), während der hartelastische Werkstoff geeigneter ist, mechanischen äußeren Beeinträchtigungen entgegen zu wirken. (Schutzfunktion).

Diese bekannte Nadelkappe erfüllt jedoch nicht nachfolgende Kriterien: a) sichere Abdichtung der Nadelspitze, b) visuelle Inspektion der Nadel bei aufgesetzter Nadelkappe möglich, c) tropfenfreies Abziehen der Nadelkappe gewährleistet, d) keine Beeinträchtigung der Nadelsilikonisierung, e) vollständige Abziehbarkeit vom Spritzenkopf ohne vorbereitende Schritte, f) Sterilisierbarkeit durch energiereiche Lichtblitze, Dampfsterilisation, g) sicheres Aufsetzen durch eindeutige und kontrollierbare Positionierung, h) sichere Wiederverschließbarkeit der Spritze nach der Applikation.

Durch die deutsche Gebrauchsmusterschrift Nr. 89 06 101 ist eine Nadelkappe mit den eingangs bezeichneten Merkmalen bekannt geworden, die auch eine sichere Abdichtung und Aufbringung der Nadelspitze gewährleistet. Die anderen vorgenannten Kriterien vermag auch diese bekannte Nadelkappe nicht zu erfüllen.

Von dieser bekannten Nadelkappe geht die Erfindung aus. Ihr liegt die Aufgabe zugrunde, diese Nadelkappe so auszubilden, daß sie auch die anderen vorgenannten Kriterien erfüllt.

Die Lösung dieser Aufgabe gelingt gemäß der Erfindung durch die Merkmale:
- die steife Kunststoffkappe besteht aus einem transparenten oder semitransparenten transluzenten Kunststoffmaterial,
- die Verbindungsanordnung weist nur eine einzige Rast-Anschlagstellung für eine kontrollierte Wegbegrenzung beim Aufstülpen der Nadelkappe auf, und
- die Dichtstrecke (A) für die Nadelspitze in der Auskleidung ist größer als die Dichtstrecke (B) der steifen Kunststoffkappe auf dem Spritzenkopf ausgebildet, derart, daß beim Abziehen der steifen Kunststoffkappe die Öffnung in der Nadelspitze erst dann von der weichelastischen inneren Auskleidung freigebbar ist, wenn der Druck im Kappeninneren dem äußeren Luftdruck entspricht.

Die erfindungsgemäße zweiteilige Nadelkappe der Einmalspritze vereinigt nachstehende Vorteile in sich:
- Die transparente Nadelkappe ermöglicht eine visuelle Inspektion der Injektionsnadel durch den Anwender auch bei aufgesetzter Nadelkappe. Die mit einem - zwecks Kontrolle - vorübergehenden Abziehen der bekannten, nicht transparenten Nadelkappe einhergehenden Beeinträchtigungen werden daher vermieden. Es ist dabei durch die DE 41 40 101 C 1 bekannt, in eine zweischalige Nadelkappe aus undurchsichtigem Material eine "Kontrollupe" einzubauen, was jedoch die Herstellung einer derartigen Nadelkappe sehr aufwendig macht.
- Die Nadelkappe ist ohne vorbereitende Schritte vollständig vom Spritzenkopf abziehbar, weder beim Aufsetzen noch beim Abziehen der Nadelkappe sind vorbereitende Schritte notwendig, zum Beispiel durch Drehen, Drücken oder Auftrennen der Nadelkappe. Es genügt ein einfaches Aufstülpen und Abziehen der Nadelkappe. Die Deformierung der Nadelkappe beim Abziehen kann die Silikonisierung der Nadel nicht beeinträchtigen.
- Die form- bzw. kraftschlüssige Verbindung (das Einrasten) der steifen äußeren Kunststoffkappe mit dem Spritzenkopf gewährleistet einen mechanisch und mikrobiologisch sicheren Schutz der äußeren Nadeloberfläche und des Spritzeninhaltes. Gemäß einer anderen Ausführung der Nadelkappe kann diese ebenfalls mit einem O-Ring zur Abdichtung auf dem Spritzenkopf ausgerüstet sein. Der in den Spritzenkopf oder in der Nadelkappe integrierte Anschlag begrenzt den Aufsetzweg bei der Nadelkappenmontage sicher. Das Einrasten der Nadelkappe am Spritzenkopf kann einfach und sicher z. B. durch akustische Detektion des Einrastgeräusches zu 100 % überprüft werden. Eine ähnliche Verbindungsanordnung ist zwar auch bei der Nadelkappe nach der Gebrauchsmusterschrift vorhanden. Diese bekannte Nadelkappe in Verbindung mit dem Spritzenkopf besitzt jedoch Verbindungselemente für zwei Rastverbindungen in zwei unterschiedlichsten Raststellungen entlang der Spritzenachse, wogegen die Verbindungsanordnung der erfindungsgemäßen Nadelkappe nur eine einzige Raststellung aufweist. Dadurch ist einmal die Ausbildung der Nadelkappe einfacher und zum anderen ist das Aufstülpen der Nadelkappe unkomplizierter, weil nicht mehr ein feinfühliges "Herantasten" an die erste Raststellung wie im bekannten Fall notwendig ist.
- Durch die Belüftung des Kappeninnenraumes, bevor die Öffnung der Nadel von der inneren Auskleidung frei gegeben ist, ist ein tropfenfreies Abziehen der Nadelkappe möglich. Dies ist bei der bekannten Nadelkappe nicht möglich. Eine Beeinflussung der zu applizierenden Dosierung sowie die mit der Benetzung der Nadeloberfläche bedingten nachteiligen Wirkungen beim Einstechen der Nadel durch die Haut eines Patienten werden vermieden, ein sehr großer Vorteil bei der Anwendung der Fertigspritzen. Die steife äußere Kunststoffkappe hat daher nicht nur eine mechanische Schutzfunktion, sondern ermöglicht erst durch ihre Formstabilität die wegabhängige Belüftung des Kappeninneren, im Gegensatz auch zu den weichelastischen bekannten Nadelkappen, bei denen sich das Kappenhemd stets, auch beim Abziehen, eng an den Spritzenkopf anschmiegt und somit eine Belüftung des Kappeninneren verhindert.
- Das Wiederaufsetzen der Nadelkappe nach Applikation wird durch die trichterförmige, konische Ausführung von Spritzenkopf und Nadelkappe wesentlich erleichtert.
- Beim Wiederaufsetzen der Nadelkappe nach dem Gebrauch der Spritze wird ein Durchstechen der Nadel, zum einen durch die mechanische Führung der Nadelkappe, zum anderen durch die Beschaffenheit der Nadelkappe, hartelastischer Kunststoff, sicher vermieden.

Gemäß einer Weiterbildung der Erfindung wird die formschlüssige Verbindung zwischen dem Spritzenkopf und der äußeren harten Kunststoffkappe durch einen Schnappverschluß gebildet. Eine derartige Ausführungsform ist verhältnismäßig einfach herzustellen und gewährleistet eine mechanisch sehr stabile und gleichzeitig mikrobiologisch sichere Abdichtung im aufgesetzten Zustand der Kunststoffkappe bei gut kontrollierbarer Positionierung der Nadelkappe. Gemäß einer weiteren Ausgestaltung der Erfindung wird der Schnappverschluß vorzugsweise in der Weise hergestellt, daß am Spritzenkopf eine rillenartige Vertiefung und an der Innenwand des Kappenhemdes ein dazu komplementärer Wulst ausgebildet sind.

Es ist gemäß einer anderen Ausgestaltung der Erfindung auch möglich, den Schnappverschluß in der Weise zu bilden, daß am Spritzenkopf eine wulst- oder alternativ eine ringbundartige Verdickung und an der Innenwand des Kappenhemdes eine komplementäre rillenartige Vertiefung ausgebildet sind. In beiden Fällen kann das Einrastgeräusch beim richtigen Aufsetzen der Nadelkappe kontrolliert und als Qualitätssicherungsmerkmal verwendet werden.

Dem Fachmann stehen hierzu verschiedene Ausführungsformen zur Verfügung, die sich auch danach bestimmen, ob der Spritzenkörper bzw. der Spritzenkopf aus Glas oder Kunststoff hergestellt ist. Kunststoffspritzen lassen sich dabei mit sehr engen Toleranzen herstellen, so daß im einfachsten Fall es für eine mechanisch und mikrobiologisch sichere formschlüssige Verbindung genügt, die Spritzenkopfkonfiguration mit sehr engen Toleranzen auf die Konfiguration des Kappenhemdes abzustimmen.

Um den Innenraum der Kunststoffkappe wegabhängig beim Abziehen der Nadelkappe zu belüften, stehen dem Fachmann eine Reihe von Möglichkeiten zur Verfügung. So ist gemäß einer Weiterbildung der Erfindung zur Belüftung des Innenraumes der Kunststoffkappe der Spritzenkopf konisch, sich zur Spitze hin verjüngend, ausgebildet.

Gemäß einer anderen Ausgestaltung der Erfindung kann alternativ dazu zur Belüftung des Innenraumes der Kunststoffkappe der Spritzenkopf eine Längsnut aufweisen, die wegabhängig frei gegeben wird. Dies ist beispielsweise möglich, wenn die Längsnut V-förmig, sich zur Spitze hin öffnend, ausgebildet ist.

Zur wegabhängigen Belüftung des Innenraumes der Kunststoffkappe kann die Anordnung auch so getroffen sein, daß zur Realisierung der unterschiedlichen Dichtstrecken die axiale Lage der Verbindungsanordnung zwischen der steifen Kunststoffkappe und dem Spritzenkopf auf dem Spritzenkopf und die axiale Ausdehnung der inneren Auskleidung der steifen Kunststoffkappe entsprechend aufeinander abgestimmt sind.

Weitere ausgestaltende Merkmale sowie Vorteile der Erfindung ergeben sich anhand der Beschreibung von in den Zeichnungen dargestellten Ausführungsbeispielen der Erfindung.

Es zeigen:
- Fig. 1: eine erste Ausführungsform der erfindungsgemäßen zweiteiligen Nadelkappe mit einer allseitig geschlossenen, steifen äußeren Kunststoffkappe,
- Fig. 2: eine weitere Ausführungsform der erfindungsgemäßen Nadelkappe mit einer äußeren steifen Kunststoffkappe, die an der Spitze eine Ausnehmung aufweist, die durch eine innere weichelastische Auskleidung verschließbar ist,
- Fig. 3: eine Nadelkappe mit einer äußeren Kunststoffkappe, entsprechend Fig. 2, die jedoch im Inneren durch eine verstärkte Wandung eine Zwangsführung für eine Injektionsnadel vorgibt,
- Fig. 4: eine erfindungsgemäße Nadelkappe, entsprechend Fig. 1, jedoch mit einem andersartig ausgebildeten Schnappverschluß,
- Fig. 5: eine weitere Ausführungsform der erfindungsgemäßen Nadelkappe mit einer äußeren Kunststoffkappe, entsprechend Fig. 1, jedoch mit einer weiteren Ausführungsform des Schnappverschlusses und einem konischen Spritzenkopf zur zwangsweise wegabhängigen Belüftung des Kappeninneren,
- Fig. 6: eine weitere Ausführungsform, ähnlich derjenigen nach Fig. 5, jedoch mit einer V-Nut im Spritzenkopf zur Zwangsbelüftung des Kappeninneren beim Abziehen,
- Fig. 7: einen Schnitt durch die Nadelkappe nach Fig. 6 entlang der dort angegebenen Schnittlinie.

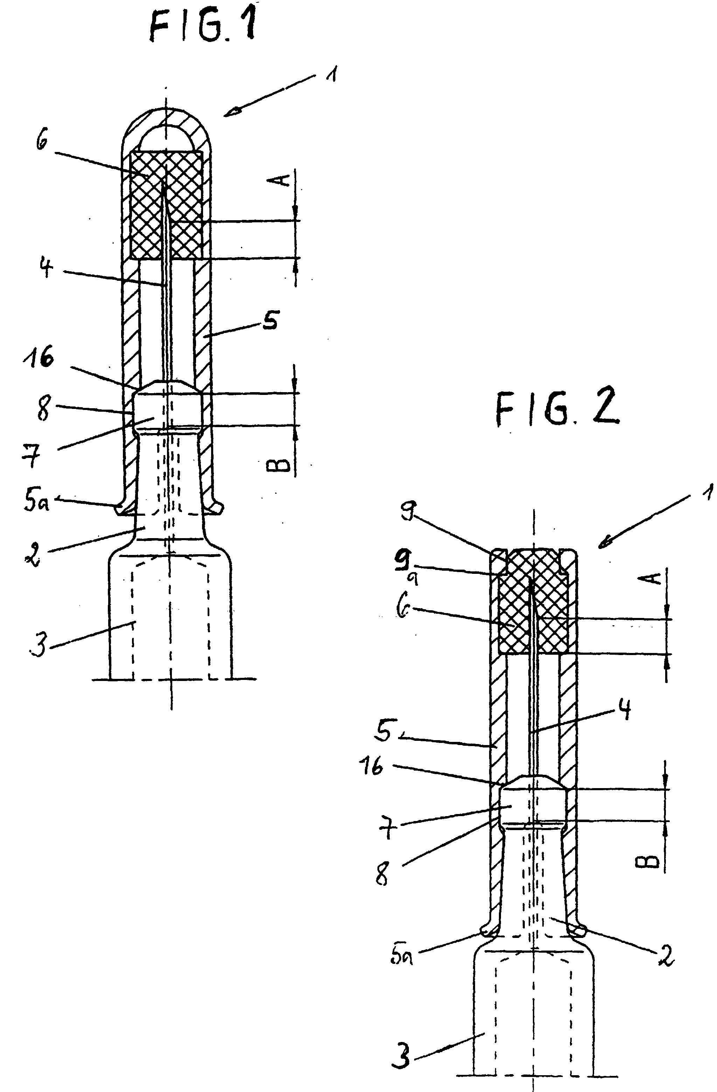

Die Fig. 1 zeigt eine generell mit 1 bezeichnete Nadelkappe für eine vorfüllbare Einmalspritze mit einer im Spritzenkopf 2 eines Spritzenkörpers 3 der Einmalspritze fest verbundenen Injektionsnadel 4. Die Nadelkappe besteht aus zwei Teilen, nämlich aus einer äußeren Kunststoffkappe 5 aus relativ hartelastischem Material, (zum Beispiel transparenten Polymeren wie Cycloolefincopolymere (COC), Polystyrol, Polyethylenterephtalat (PET), Polyethylennaphtylat (PEN), Polymethylpenten (TP), Acrylbutadienstyrol (ABS), Polycarbonat (PC) oder semitransparenten Polymeren, wie Polyethylen, Polypropylen) die innen im Bereich der Nadelspitze mit einer aus einem weichelastischen Werkstoff z. B. Naturkautschuk, Halogenbuthylelastomere, vorzugsweise einem transluzenten gummiartigen Elastomer (z. B. Silikonkautschuk, thermoplastische Elastomere, COC-Elastomere), bestehenden dichtenden Auskleidung 6 versehen ist und einen umlaufenden Einführkonus 5a zur Erleichterung des Wiederaufsetzens der Nadelkappe nach der Injektion besitzt. Die Auskleidung 6 ist dabei formschlüssig in einer entsprechenden Ausnehmung der Kunststoffkappe 5 aufgenommen. In einer weiteren Ausführungsform ist es möglich, mittels einem Zweikomponentenspritzgußverfahrens die äußere Hülle aus einem der o.g. Kunststoffe mit einem weich-elastischen thermoplastischen Elastomer (TPE) oder Silikonkautschuk in einem Arbeitsschritt herzustellen. Die Nadelspitze wird durch die Auskleidung 6 abgedichtet, indem die Nadel in dieses Teil eingestochen wird.

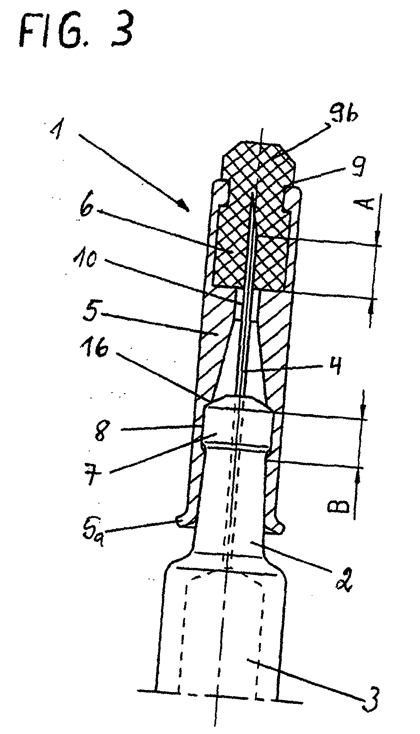

Die Nadelkappe 1 ist dabei ohne vorbereitende Schritte auf dem Spritzenkopf 2 aufstülpbar und von ihm abziehbar. Gemäß einem ersten Merkmal der Erfindung besteht die Kunststoffkappe 5 aus einem transparenten Kunststoffmaterial, wodurch mit Vorteil eine visuelle Inspektion der Injektionsnadel bei aufgesetzter Nadelkappe möglich ist. Gemäß einem weiteren Merkmal der Erfindung besitzen der Spritzenkopf 2 und die Kunststoffkappe 5 Elemente einer mechanisch stabilen formschlüssigen Verbindung bei gleichzeitig mikrobiologisch sicheren Abdichtung im aufgesetzten Zustand der Kunststoffkappe 5. Bei der Ausführungsform nach der Fig. 1, ebenso wie bei den Ausführungsformen nach den Figuren 2 und 3, wird diese formschlüssige Verbindung durch einen Schnappverschluß in der Weise gebildet, daß an dem Spritzenkopf 2 eine ringbundartige Verdickung 7 und an der Innenwand des Kappenhemdes eine komplementäre Ausnehmung 8 ausgebildet sind. Der Anschlag 16 an der Ausnehmung 8 sorgt dabei für eine kontrollierbare Wegbegrenzung beim Aufstülpen der Nadelkappe.

Gemäß einem weiteren Merkmal der Erfindung sind der Spritzenkopf 2 und die Nadelkappe 1 so aufeinander abgestimmt ausgebildet, daß beim Abziehen der Kunststoffkappe mit der inneren Auskleidung 6 die Öffnung in der Nadelspitze erst dann von der weichelastischen inneren Auskleidung 6 der Kunststoffkappe 5 frei gegeben wird, wenn das Kappeninnere zur Herstellung eines Druckausgleiches belüftet ist, d. h. der Druck im Kappeninneren dem äußeren Luftdruck entspricht. Dies erfolgt bei der Ausführungsform nach der Fig. 1, ebenso wie bei den Ausführungsformen nach den Figuren 2 bis 4 dadurch, daß die axiale Lage der formschlüssigen Verbindung 7, 8 und die axiale Ausdehnung der inneren Auskleidung 6 der Kunststoffkappe 5 so aufeinander abgestimmt sind, daß beim Abziehen der Nadelkappe 1 das Kappeninnere zwangsbelüftet ist, bevor die Öffnung in der Nadelspitze von der inneren Auskleidung 6 freigegeben ist. Konstruktiv wird das tropfenfreie Abziehen dadurch gewährleistet, daß die Dichtstrecke A für die Nadelspitze größer ist als die Dichtstrecke B der Nadelkappe auf dem Spritzenkopf. Dadurch ist, wie eingangs erwähnt, mit großem Vorteil ein tropfenfreies Abziehen der Nadelkappe 1 möglich.

Bei der Ausführungsform nach der Fig. 1 ist die äußere Kunststoffkappe 5 allseitig geschlossen. Die Einbringung der inneren weichelastischen Auskleidung 6, die vorzugsweise aus einem gummiartigen Elastomer besteht, ist daher nur spritzenkopfseitig über die zugehörige Öffnung in der äußeren Kunststoffkappe 5 möglich.

Bei der Ausführungsform nach der Fig. 2, die im übrigen mit derjenigen nach Fig. 1 übereinstimmt, wobei gleiche Teile mit denselben Bezugszeichen versehen sind, weist jedoch die äußere Kunststoffkappe 5 an der Spitze eine Ausnehmung 9 auf, die durch die innere weichelastische Auskleidung 6 dicht verschließbar ist. Bei der Ausführungsform nach Fig. 2 schließt dabei der obere Rand der inneren Auskleidung 6 bündig mit der Öffnung 9 ab, wobei diese innere Auskleidung an unteren Schultern 9a der Ausnehmung gehalten ist. Die Ausführungsform nach Fig. 2 ermöglicht dabei eine Montage der inneren Auskleidung 6 von der Kappenspitze her, was gegebenenfalls fertigungstechnisch günstiger sein kann als die Montageart nach Fig. 1.

Die Fig. 3 zeigt eine weitere Ausführungsform entsprechend Fig. 2 mit folgenden Unterschieden. Die innere Auskleidung 6 schließt nicht bündig mit der Öffnung 9 ab, sondern steht etwas darüber hinaus. Dadurch wird die Montage der Nadelkappe aus ihren beiden Komponenten erleichtert, beispielsweise dadurch, daß die innere Auskleidung an ihrem überstehenden Ende 9b sicher gefaßt werden kann.

Als Dichtstrecken sind die maximalen Strecken A, B bezeichnet, über die die Nadelkappe abgezogen werden kann, ohne daß die Abdichtung der Nadelöffnung bzw. die Verbindung Spritzenkopf - Nadelkappe aufgehoben wird.

Die innere weichelastische Auskleidung 6 wird dabei pfropfenartig in der Ausnehmung 9 gehaltert. Ferner weist die Ausführungsform nach Fig. 3 durch eine verstärkte Wandung der äußeren Kunststoffkappe 5 eine Zwangsführung 10 für die Injektionsnadel 4 auf, was die Handhabung beim Aufstülpen der Nadelkappe 1 erleichtert.

In den Ausführungsformen mit oben offener Nadelkappe (Fig. 2 + 3) ist es möglich, die Nadel nach dem Aufsetzen der Nadelkappe durch eine Dampfsterilisation zu sterilisieren, da der weichelastische Teil leicht von Dampf durchdrungen werden kann. Sollte eine zusätzliche dampfoffene Entlüftung der Nadelkappe am Spritzenkopf notwendig sein, kann dies durch eine Labyrinthdichtung gewährleistet werden. Diese Labyrinthdichtung ermöglicht einen Durchtritt von Wasserdampf während der Dampfsterilisation, dichtet jedoch mikrobiologisch sicher ab. Die Labyrinthdichtung ist in Fig. 1 - 7 nicht gezeigt.

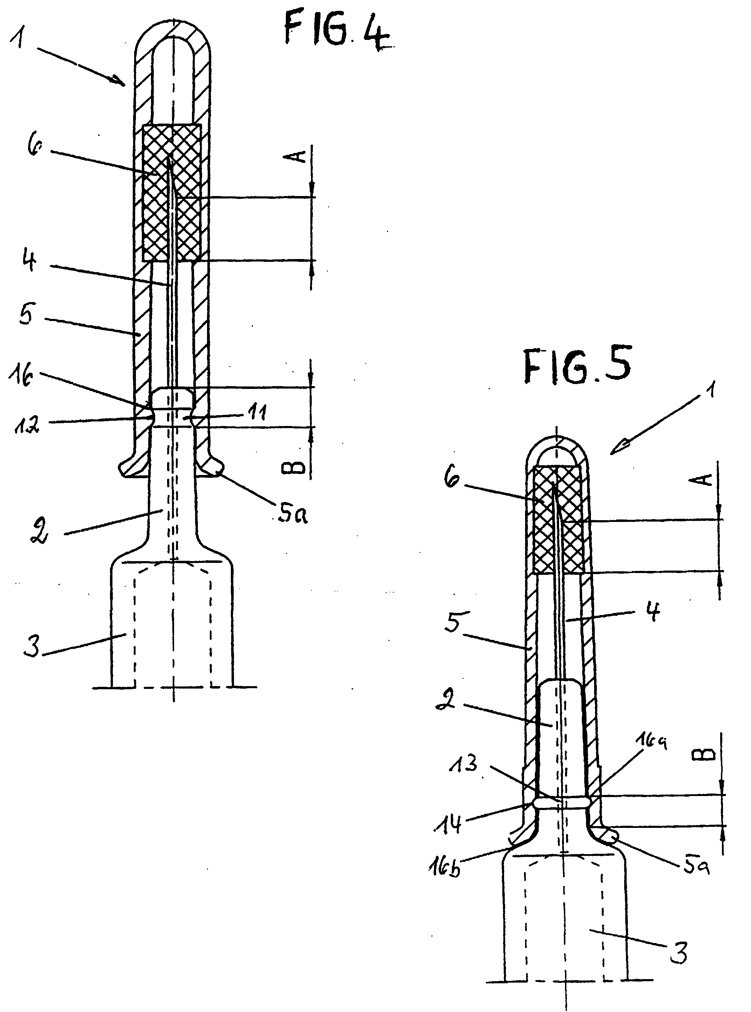

Die Fig. 4 zeigt eine Ausführungsform der erfindungsgemäßen Nadelkappe ähnlich derjenigen nach Fig. 1, jedoch mit einer anderen Ausführungsform eines Schnappverschlusses. Dieser wird im Fall der Ausführungsform nach Fig. 4 dadurch realisiert, daß am Spritzenkopf 2 eine rillenartige umlaufende Vertiefung 11 und an der Innenwand des Kappenhemdes der Kunststoffkappe 5 ein dazu komplementärer Ringwulst 12 ausgebildet sind. Im übrigen gelten die zu den anderen Ausführungsformen bisher gemachten Ausführungen auch für die Gestaltung der Nadelkappe nach Fig. 4 entsprechend.

Die Fig. 5 zeigt ebenfalls wie die Fig. 4 eine Ausführungsform der erfindungsgemäßen Nadelkappe entsprechend derjenigen nach Fig. 1, bei der jedoch eine weitere Variante sowohl für den Schnappverschluß als auch für die wegabhängige Zwangsentlüftung dargestellt sind. Bei der Ausführungsform nach Fig. 5 wird die formschlüssige Verbindung in Form des Schnappverschlusses durch eine ringwulstartige Verdickung 13 und eine dazu komplementäre Ausnehmung 14 an der Innenwand des Kappenhemdes der Kunststoffkappe 5 gebildet, wobei die Kunststoffkappe 5 im unteren Bereich zur Erhalt der mechanischen Stabilität eine etwas verstärkte Wanddicke aufweist. Im Gegensatz zu den bisherigen Ausführungsformen ist der Spritzenkopf 2 bei der Ausführungsform nach Fig. 5 konisch, sich zur Spitze hin verjüngend, ausgebildet, so daß über diese Konizität des Spritzenkopfes beim Abziehen der Nadelkappe eine zwangsweise Belüftung des Kappeninneren erfolgt. Die Steifigkeit der Kunststoffkappe 5 verhindert dabei, daß sich diese an die Konizität des Spritzenkopfes 2 anpaßt, um so die wegabhängige Zwangsbelüftung zu ermöglichen. Anschläge 16a und 16b sorgen für eine kontrollierte Wegbegrenzung beim Aufstülpvorgang.

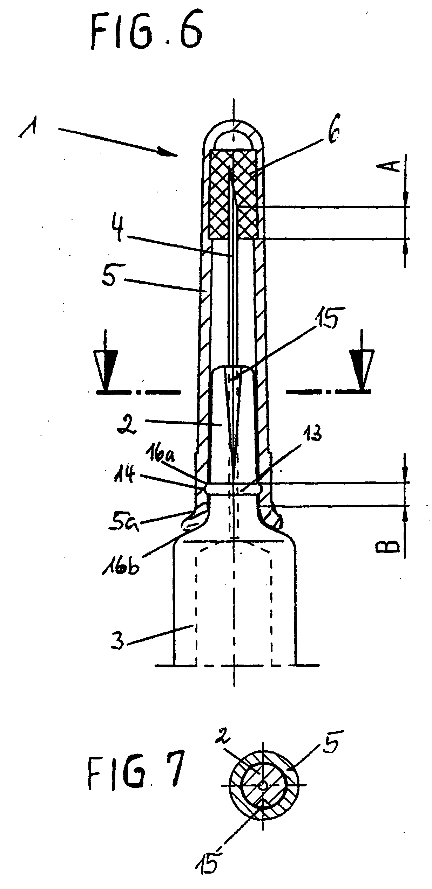

Die Fig. 6 mit der Schnittansicht nach Fig. 7 schließlich zeigt eine Ausführungsform entsprechend derjenigen nach Fig. 5, jedoch mit dem Unterschied, daß der Spritzenkopf 2 eine V-förmige Längsnut 15 aufweist, die sich zur Spitze hin öffnet, und die beim Abziehen der Kappe 5 unter entsprechender Zwangsbelüftung des inneren der Kappe progressiv wirksam wird und so ein tropfenfreies Abziehen der Nadelkappe 1 ermöglicht.

## Patentansprüche

1. Einmalspritze mit einer im Spritzenkopf (2) fest verbundenen Injektionsnadel (4), und einer Nadelkappe (1), bestehend aus einer äußeren steifen Kunststoffkappe (5) aus hartelastischem Material, die innen, zumindest im Bereich der Nadelspitze, mit einer aus einem weichelastischen Werkstoff bestehenden dichtenden Auskleidung (6) versehen ist, und die einfach auf den Spritzenkopf (2) aufstülpbar und ohne vorbereitenden Schritt von ihm vollständig abziehbar ist, wobei der Spritzenkopf (2) und die steife Kunststoffkappe (5) Elemente (7, 8; 11, 12; 13, 14) einer mechanisch stabilen form- und/oder kraftschlüssigen, den Aufstülpweg begrenzenden, Verbindungsanordnung besitzen bei gleichzeitig mikrobiologisch sicheren Abdichtung im aufgesetzten Zustand der steifen Kunststoffkappe, **gekennzeichnet durch** die Merkmale:
- die steife Kunststoffkappe (5) besteht aus einem transparenten oder semitransparenten transluzenten Kunststoffmaterial,
- die Verbindungsanordnung weist nur eine einzige Rast-Anschlagstellung für eine kontrollierte Wegbegrenzung beim Aufstülpen der Nadelkappe auf, und
- die Dichtstrecke (A) für die Nadelspitze in der Auskleidung (6) ist größer als die Dichtstrecke (B) der steifen Kunststoffkappe (5) auf dem Spritzenkopf (2) ausgebildet derart, daß beim Abziehen der Kunststoffkappe (5) die Öffnung in der Nadelspitze erst dann von der weichelastischen inneren Auskleidung (6) freigebbar ist, wenn der Druck im Kappeninneren dem äußeren Luftdruck entspricht.

2. Einmalspritze nach Anspruch 1, **dadurch gekennzeichnet, daß** die form- und/oder kraftschlüssige Verbindung durch einen Schnappverschluß (7, 8; 11, 12; 13, 14) gebildet ist.

3. Einmalspritze nach Anspruch 2, **dadurch gekennzeichnet, daß** am Spritzenkopf (2) eine rillenartige umlaufende Vertiefung (11) und an der Innenwand des Kappenhemdes ein dazu komplementärer Ringwulst (12) ausgebildet sind.

4. Einmalspritze nach Anspruch 2, **dadurch gekennzeichnet, daß** am Spritzenkopf (2) eine ringwulst- oder alternativ eine ringbundartige Verdickung (7, 13) und an der Innenwand des Kappenhemdes eine dazu komplementäre Ausnehmung (8, 14) ausgebildet sind.

5. Einmalspritze nach einem der Ansprüche 1 bis 4, **dadurch gekennzeichnet, daß** zur Belüftung des Innenraumes der steifen Kunststoffkappe (5) der Spritzenkopf (2) konisch, sich zur Spitze hin verjüngend, ausgebildet ist.

6. Einmalspritze nach einem der Ansprüche 1 bis 4, **dadurch gekennzeichnet, daß** zur Belüftung des Innenraumes der steifen Kunststoffkappe (5) der Spritzenkopf (2) eine Längsnut (15) aufweist.

7. Einmalspritze nach Anspruch 6, **dadurch gekennzeichnet, daß** die Längsnut (15) V-förmig, sich zur Spitze hin öffnend, ausgebildet ist.

8. Einmalspritze nach einem der Ansprüche 1 bis 4, **dadurch gekennzeichnet, daß** zur Realisierung der unterschiedlichen Dichtstrecken die axiale Lage der Verbindungsanordnung (7, 8; 11, 12; 13, 14) zwischen der steifen Kunststoffkappe (5) und dem Spritzenkopf (2) auf dem Spritzenkopf und die axiale Ausdehnung der inneren Auskleidung (6) der steifen Kunststoffkappe entsprechend aufeinander abgestimmt sind.

9. Einmalspritze nach einem der Ansprüche 1 bis 8, **dadurch gekennzeichnet, daß** die äußere steife Kunststoffkappe (5) einen allseitig geschlossenen Kappenmantel aufweist.

10. Einmalspritze nach einem der Ansprüche 1 bis 8, **dadurch gekennzeichnet, daß** die äußere steife Kunststoffkappe (5) an der Spitze eine Ausnehmung (9) aufweist, die durch die innere weichelastische Auskleidung (6) dicht verschließbar ist.

11. Einmalspritze nach einem der Ansprüche 1 bis 10, **dadurch gekennzeichnet, daß** die steife Kunststoffkappe (5) in ihrem Inneren eine Zwangsführung (10) für die Injektionsnadel (4) aufweist.

## Claims

1. Disposable syringe with an injection needle (4) firmly secured in the syringe head (2), and with a needle cap (1) consisting of an outer stiff plastic cap (5) which is made of hard-elastic material and which is provided on the inside, at least in the area of the needle tip, with a sealing lining (6) made of a soft-elastic material, and which can be easily pushed onto the syringe head (2) and can be completely removed from the latter without any preparatory step, the syringe head (2) and the stiff plastic cap (5) comprising elements (7, 8; 11, 12; 13, 14) of a mechanically stable form-fitting and/or positive-locking connection arrangement which limits the push-on distance and at the same time provides for microbiologically reliable sealing when the stiff plastic cap is fitted, **characterized by** the following features:
- the stiff plastic cap (5) consists of a transparent or semi-transparent translucent plastic material,
- the connection arrangement has only one locking stop position for controlled limitation of the push-on distance when fitting the needle cap, and
- the sealing distance (A) for the needle tip in the lining (6) is greater than the sealing distance (B) of the stiff plastic cap (5) on the syringe head (2) so that, when the plastic cap (5) is removed, the opening in the needle tip can be released from the soft-elastic inner lining (6) only when the pressure in the cap interior corresponds to the external air pressure.

2. Disposable syringe according to Claim 1, **characterized in that** the form-fitting and/or positive-locking connection is formed by a snap coupling (7, 8; 11, 12; 13, 14).

3. Disposable syringe according to Claim 2, **characterized in that** a groove-like circumferential depression (11) is formed on the syringe head (2), and a complementary annular bead (12) is formed on the inner wall of the cap skirt.

4. Disposable syringe according to Claim 2, **characterized in that** a thickened portion (7, 13) in the form of an annular bead or alternatively in the form of an annular collar is formed on the syringe head (2), and a complementary recess (8, 14) is formed on the inner wall of the cap skirt.

5. Disposable syringe according to one of Claims 1 to 4, **characterized in that**, in order to aerate the interior of the stiff plastic cap (5), the syringe head (2) is designed tapering conically towards the tip.

6. Disposable syringe according to one of Claims 1 to 4, **characterized in that** the syringe head (2) has a longitudinal groove (15) for aerating the interior of the stiff plastic cap (5).

7. Disposable syringe according to Claim 6, **characterized in that** the longitudinal groove (15) is designed in a V-shape which opens towards the tip.

8. Disposable syringe according to one of Claims 1 to 4, **characterized in that**, in order to realize the different sealing distances, the axial position of the connection arrangement (7, 8; 11, 12; 13, 14) between the stiff plastic cap (5) and the syringe head (2) on the syringe head and the axial extent of the inner lining (6) of the stiff plastic cap are correspondingly adapted to one another.

9. Disposable syringe according to one of Claims 1 to 8, **characterized in that** the outer stiff plastic cap (5) has a cap jacket which is closed all round.

10. Disposable syringe according to one of Claims 1 to 8, **characterized in that** the outer stiff plastic cap (5) has, at the tip, a recess (9) which can be closed off tightly by the inner soft-elastic lining (6).

11. Disposable syringe according to one of Claims 1 to 10, **characterized in that** the stiff plastic cap (5) has, in its interior, a constraining guide (10) for the injection needle (4).

## Revendications

1. Seringue à usage unique, comprenant une aiguille d'injection (4) connectée fixement dans la tête de la seringue (2), et un capuchon d'aiguille (1), se composant d'un capuchon en plastique rigide extérieur (5) en matériau élastique dur, qui est pourvu à l'intérieur, au moins dans la région de la pointe de l'aiguille, d'un revêtement (6) d'étanchéité, se composant d'un matériau élastique mou, et qui peut être simplement enfoncé sur la tête de la seringue (2) et retiré complètement de celle-ci sans étape préparatoire, la tête de la seringue (2) et le capuchon en plastique rigide (5) possédant des éléments (7, 8 ; 11, 12 ; 13, 14) d'un agencement de liaison stable mécaniquement, à engagement positif et/ou par friction, limitant la course d'enfoncement, pour une fermeture étanche en même temps plus sûre du point de vue microbiologique dans l'état posé du capuchon en plastique rigide, **caractérisée par** les caractéristiques suivantes :
- le capuchon en plastique rigide (5) se compose d'un matériau en plastique translucide transparent ou semi-transparent,
- l'agencement de liaison présente seulement une position de butée d'encliquetage unique pour une limitation contrôlée de la course lors de l'enfoncement du capuchon d'aiguille, et
- l'extension d'étanchéité (A) pour la pointe d'aiguille dans le revêtement (6) est plus grande que l'extension d'étanchéité (B) du capuchon en plastique rigide (5) sur la tête de la seringue (2), de telle sorte que lors du retrait du capuchon en plastique (5), l'ouverture dans la pointe d'aiguille puisse seulement être libérée par le revêtement intérieur élastique mou (6) lorsque la pression à l'intérieur du capuchon correspond à la pression de l'air extérieur.

2. Seringue à usage unique selon la revendication 1, **caractérisée en ce que** la liaison par engagement positif et/ou par friction est formée par une fermeture par encliquetage (7, 8 ; 11, 12 ; 13, 14).

3. Seringue à usage unique selon la revendication 2, **caractérisée en ce qu'**un renfoncement périphérique en forme de gorge (11) est réalisé sur la tête de la seringue (2) et un bourrelet annulaire (12) complémentaire à celui-ci est réalisé sur la paroi intérieure de la chemise du capuchon.

4. Seringue à usage unique selon la revendication 2, **caractérisée en ce qu'**un épaississement de type bourrelet annulaire ou en variante de type épaulement annulaire (7, 13) est réalisé sur la tête de la seringue (2), et un évidement (8, 14) complémentaire à celui-ci est réalisé sur la paroi intérieure de la chemise du capuchon.

5. Seringue à usage unique selon l'une quelconque des revendications 1 à 4, **caractérisée en ce que** pour l'aération de l'espace interne du capuchon en plastique rigide (5), la tête de la seringue (2) est réalisée avec une forme conique se rétrécissant vers la pointe.

6. Seringue à usage unique selon l'une quelconque des revendications 1 à 4, **caractérisée en ce que** pour l'aération de l'espace interne du capuchon en plastique rigide (5), la tête de la seringue (2) présente une rainure longitudinale (15).

7. Seringue à usage unique selon la revendication 6, **caractérisée en ce que** la rainure longitudinale (15) est réalisée en forme de V, en s'ouvrant vers la pointe.

8. Seringue à usage unique selon l'une quelconque des revendications 1 à 4, **caractérisée en ce que** pour la réalisation des extensions d'étanchéité différentes, la position axiale de l'agencement de liaison (7, 8 ; 11, 12 ; 13, 14) entre le capuchon en plastique rigide (5) et la tête de la seringue (2) sur la tête de la seringue et l'étendue axiale du revêtement intérieur (6) du capuchon en plastique rigide sont adaptées de manière correspondante l'une à l'autre.

9. Seringue à usage unique selon l'une quelconque des revendications 1 à 8, **caractérisée en ce que** le capuchon en plastique rigide extérieur (5) présente une enveloppe de capuchon fermée de tous les côtés.

10. Seringue à usage unique selon l'une quelconque des revendications 1 à 8, **caractérisée en ce que** le capuchon en plastique rigide extérieur (5) présente un évidement (9) sur la pointe, qui peut être fermé de manière étanche par le revêtement (6) intérieur élastique mou.

11. Seringue à usage unique selon l'une quelconque des revendications 1 à 10, **caractérisée en ce que** le capuchon en plastique rigide (5) présente dans son intérieur un guidage forcé (10) pour l'aiguille d'injection (4).
